Europäisches Patentamt

European Patent Office

Office européen des brevets

(11) Publication number: **0 109 856**
**A2**

(12) # EUROPEAN PATENT APPLICATION

(21) Application number: **83307119.4**

(22) Date of filing: **21.11.83**

(51) Int. Cl.³: **G 01 N 33/76**
**G 01 N 33/54**

(30) Priority: **22.11.82 US 443688**

(43) Date of publication of application:
**30.05.84 Bulletin 84/22**

(84) Designated Contracting States:
**DE FR GB IT**

(71) Applicant: **MONOCLONAL ANTIBODIES, INC.**
**2319 Charleston Road**
**Mountain View California 94043(US)**

(72) Inventor: **Danisch, Robert J.**
**6035 Birch Place**
**Newark California 94560(US)**

(74) Representative: **Bizley, Richard Edward et· al,**
**BOULT, WADE & TENNANT 27 Furnival Street**
**London EC4A 1PQ(GB)**

(54) **HCG sandwich-type immunoassay and reducing LH cross-reactivity in an immunological reaction for HCG with LH present.**

(57) A method for reducing the cross-reactivity of LH when analyzing for hCG in a biologically derived fluid sample which includes both hCG and LH. The technique may be a conventional sandwich-type immunoassay in which the hCG is bound to immobilized antibody and to free labeled antibody, to form a sandwich. The binding to the two different antibodies may be performed either simultaneously or sequentially. The improvement involves the binding of the labeled antibody in a buffered solution at a limited concentration of salt, typically sodium chloride, namely, to a maximum concentration of 0.5 weight/volume % of the salt, to reduce the cross-reactivity with LH. Reduction in LH cross-reactivity is also assisted by reducing the buffering agent concentration.

HCG SANDWICH-TYPE IMMUNOASSAY AND REDUCING LH CROSS-
REACTIVITY IN AN IMMUNOLOGICAL REACTION FOR HCG WITH
LH PRESENT

Monoclonal antibodies are highly useful for the
analysis of various glycoprotein hormones such as
human choriogonadotropin (hCG). hCG is analyzed from
biologically derived samples, e.g., derived from
urine or serum. A particularly effective technique
is known as the two-site sandwich methodology, using
enzyme labels as set forth in H.G. Wada et al., Clin.
Chem. 28/9, 1862-1866 (1982). The anti-alpha-subunit
monoclonal antibody is used as the solid-phase (e.g.,
a coated tube) to capture the hCG. Luteinizing
hormone (LH) may be present at significant concentrations in serum or urine. During incubation, the
immobilized antibody captures both the hCG and LH.
Anti-beta subunit monoclonal antibodies are coupled
to a label, such as alkaline phosphatase or
horseradish peroxidase, and reacted with the bound
hCG and LH on the solid surface. The free labeled
antibody is separated from the bound antibody, and
the label on the latter is detected. In the Wada et
al article, this technique is performed in a
so-called simultaneous sandwich technique, i.e.,
binding of the hCG to the immobilized antibody and to
the labeled antibody are performed in a single
incubation step. The sandwich technique is also
known to be performed sequentially so that the serum
or urine sample is first incubated with the solid

phase (immobilized antibody), the soluble sample is removed, and then the solid phase is immunologically reacted with the labeled antibody. One problem with the above sandwich technique is the cross-reactivity of LH in the tested sample for the labeled antibody. Thus, as set forth in the above Wada et al paper, cross-reactivity was shown to be 8.3% for LH at μg/L or 200 international units/L (IU/L).

In accordance with the present invention, cross-reactivity for LH in a sandwich-type immunoassay for hCG is significantly reduced by controlling the maximum salt content of the buffered solution utilized during reaction of the labeled antibody with the hCG. The maximum salt content is on the order of 0.5 weight/volume % in the buffered solution, in comparison to a standard buffered solution containing about 0.9 weight/volume %.

The present invention relates to a sandwich-type of immunoassay for hCG in a biologically-derived fluid, typically derived from serum or urine.

In the sandwich-type of immunoassay to which the present invention is applicable, monoclonal antibody to the alpha-subunit of hCG and LH is first immobilized onto a solid surface. Then, it is contacted with the serum or urine, and the hCG and accompanying LH in the sample is bound to the immobilized antibody. Anti-beta-subunit monoclonal antibodies coupled to a label, preferably an enzyme, is contacted with the hCG and bound thereto. Then, the free labeled antibody is removed and the bound labeled antibody is detected as a measure of the quantity of hCG in the fluid sample. The two incubation steps may be performed sequentially for either serum or urine samples. On the other hand, the simultaneous technique may be utilized for urine sample, but preferably not for a serum sample. This is because other proteins in the serum sample can cause a non-specific binding problem for the enzyme labeled antibody.

In the first step, the antibody is immobilized by binding to a solid surface such as a tube, stick, beads, plate, paper, a disk or the like. Typically, the surface to be coated is the interior of a test tube or the end of a dip stick, convenient surfaces for coating and washing.

One suitable surface for bonding of antibody is a plastic material such as polystyrene, polypropylene, or the like. Other plastic materials may also be employed. The advantage of plastic such as polystyrene is that relatively strong bonds are formed by adsorption. Antibody may also be covalently bonded to the above plastic materials or other solid materials. The use of such covalent bonding techniques is described in Biochemical Aspects of Reactions on Solid Supports, G. R. Stark, Ed., Academic Press, 1970.

In a typical instance, the antibody to be immobilized is dissolved in a suitable buffer (e.g., phosphate buffered isotonic saline) at a suitable pH. A suitable antibody concentration is between 0.5 and 50 µg/ml. An aliquot of 0.5 ml of antibody solution is placed into the bottom of a test tube formed, for example, of polystyrene or polypropylene. The filled tubes are incubated at room temperature for 4 to 48 hours. After adsorption, excess antibody is removed such as by washing in a suitable wash solution.

As set forth above, the present invention is applicable to either a simultaneous or a sequential type of immunoassay method for hCG. Referring to the sequential assay, the biologically derived sample (urine or serum) which includes hCG and LH, is contacted with the immobilized anti-alpha-subunit monoclonal antibody bound in a solid layer to a solid surface. For simplicity of description, the present specification will refer to the immobilized antibody as a layer on the inside surface of a test tube. The sample is incubated in the test tube until the hCG and LH in the sample are immunologically bound to the antibody to form a solid phase of immobilized antibody immunologically bound to hCG and LH (referred to herein as antibody-(hCG and LH). Then, the sample solution is removed from the test tube, typically followed by one or more washing steps.

Thereafter, free labeled anti-beta-subunit monoclonal antibody, specific for the beta-subunit of hCG but also reactive with LH, is added to the test tube in a buffered solution of a precise maximum quantity of a salt which includes potassium chloride, sodium chloride, or both. The mixture is then incubated to cause an immunological reaction, to thereby form a sandwich of immobilized antibody-(hCG and LH)-labeled antibody. The reaction mixture containing the free labeled antibody is then separated from the bound labeled antibody on the solid surface, and the bound labeled antibody is detected as a measure of the quantity of hCG in the fluid sample.

Control of cross-reactivity for LH in the fluid sample by controlling a character of the buffered solution in which the labeled antibody is present during incubation is a major feature of the present invention. A conventional solution for the labeled antibody is an isotonic buffered solution with a salt concentration of 0.9 weight/volume % sodium chloride. At such concentrations, it has been found that there is significant cross-reactivity for LH. Cross-reactivity is determined by assaying for LH in the absence of hCG. Cross-reactivity is defined in the following equation.

$$\% \text{ Cross-Reactivity} = \frac{\text{LH assay concentration}}{\text{LH actual concentration}} \times 100$$

By reducing the salt concentration, LH cross-reactivity is reduced. One problem in reducing cross-reactivity for the LH is that there is a loss in the detection of a certain amount of hCG in the sample. However, this loss in reactivity of the hCG is far less than the loss in reactivity of the LH. Thus, the preferred buffer solution is one that provides an acceptable minimum reduction in the detection of the hCG, while maximizing the loss in cross-reactivity of LH. In that regard, it has been found that a preferred concentration of buffering agent is in the 40 to 60 mM range, with a preferred salt concentration of the order of 0.1 weight/volume %.

It has been found that, for typical buffer solutions, the preferred salt concentration is from 0.05 to 0.5 weight/volume % salt. This is substantially lower than the conventional 0.9% sodium chloride used in a conventional buffered solution.

The concentration of buffering agent in the buffered solution also has an effect upon the amount of cross-reactivity. Thus, in a typical buffer solution of 10 mM tris, the cross-reactivity drops to a significantly lower level than in a 50 mM tris buffer solution. When the concentration of buffering agent is increased, say to 100 mM, the cross-reactivity significantly increases. Thus, it is preferable that the buffering agent be present at a concentration no greater than 100 mM. Any conventional buffering agent for this system may be utilized, such as phosphate (e.g., sodium phosphate), tris (e.g., tris-MgCl$_2$ buffer), glycine, and histidine borate.

As set forth above, the present invention is also applicable to the simultaneous immunoassay technique as well as the above-described sequential technique. There, the sample and labeled antibody are added simultaneously to the tube containing the immobilized antibody to form the above-described sandwich. Thereafter, the free labeled antibody and the sample are separated from the test tube and the bound labeled antibody on the tube is detected as a measure of the quantity of hCG in the fluid sample. The limits of concentration of the salt and buffering agent are calculated on the basis of the solution of labeled antibody in the absence of the sample. A suitable general technique for performing simultaneous sandwich-type immunoassay in accordance with the present invention is described in the aforementioned Wada et al article, with the exception that the specific buffer solution of the present invention is substituted for the one described in that paper.

Any of the known techniques may be employed to irreversibly bind the label, preferably enzyme, to the free antibody for use in the present invention. See, for example, the Periodate method described in Wilson, M. B., et al, in Immuno-fluorescence and Related Staining Techniques; W. Knapp, et al, Eds.,

Elsevier-North Holland Biomedical Press, New York-Amsterdam, 1978, pp. 215-224.

The enzyme activity of the solid phase is determined by incubation with a substrate and measuring the reaction.

Although the present system is described using enzyme as the preferred label, it should be understood that the same principles apply to other labels such as of the radioactive, fluorometric, or colorometric type.

A further disclosure of the nature of the present invention is provided by the following specific examples of its practice. It should be understood that the data disclosed serves only to exemplify and is not intended to limit the scope of the invention. Percentage concentrations are on a weight/volume basis. Percent cross-reactivity is abbreviated as "% CRX".

Example 1 (Sequential Sandwich Immunoassay for hCG)

A polystyrene tube is coated with anti-alpha-subunit monoclonal antibody specific for the alpha chain of hCG and LH (clone A57B5). hCG is partially purified for pregnancy urine and calibrated against WHO 1st IRP no. 75/537. LH is calibrated against WHO 1st IRP 68/40. The system is incubated to bind LH and hCG to the walls of the tube.

Excess sample is then washed with a solution of normal saline containing surfactant. After washing, a specific monoclonal antibody for the beta chain of the hCG (clone 5004) is added. The antibody is covalently coupled to the enzyme alkaline phosphatase. The antibody-enzyme conjugate is added in a tris buffer solution containing 1 mg/ml bovine serum albumin, a surfactant (0.04% Tween-20) and sodium chloride at concentrations of 0%, 0.1%, and 0.9%. Unbound, or free, antibody-enzyme conjugate is washed away with a saline solution containing surfactant as set forth above. The enzyme substrate BCIP (5-bromo, 4-chloro, 3-indolyl-phosphate) is then added in a tris-$MgCl_2$ buffer, at a concentration of 1 mg/ml and a pH of 9.8, and allowed to incubate until a blue color develops. The enzyme reaction is then stopped with a solution of 10mM EDTA, and read in a spectrophotometer at 620 nm.

The results of two different runs using the above procedure are set out in Table 1.

### TABLE 1

|  | Run 1 | | Run 2 | |
|---|---|---|---|---|
| Na Cl Conc. | 0.0% | 0.9% | 0.1% | 0.9% |
| OmIU/ml (hCG) | 0.007 | 0.009 | 0.026 | 0.025 |
| 50mIU/ml (hCG) | 0.056 | 0.108 | 0.100 | 0.119 |
| 400mIU/ml (LH) | 0.010 | 0.109 | 0.056 | 0.151 |
| % CRX | 0.8% | 12.5% | 5.3% | 16.8% |

Example 2

The above procedure was utilized varying the sodium chloride concentration sequentially from at 0.9%, 0.45%, 0.1%, 0.045% for tris-buffer solutions at 10 mM, 50 mM, and 100 mM. The results are set forth in Table 2 on the following page.

Example 3

The procedure of Example 2 is followed with the exception that potassium chloride was substituted for sodium chloride. The results are set forth in Table 3 on the following page.

It is apparent from Example 3 that, as the salt concentration is decreased within the above ranges, so does the LH cross-reactivity. This holds true for potassium chloride or sodium chloride salt at the different concentrations of tris buffer. Although the decrease is present at these different concentrations, it is also apparent that the cross-reactivity is higher for higher buffer content, but the detectibility of the hCG remains at a more constant high level.

## TABLE 2

| | 10 mM Tris | | | | | 50 mM Tris | | | | | 100 mM Tris | | | |
|---|---|---|---|---|---|---|---|---|---|---|---|---|---|---|
| NaCl Concentration (%) | .9 | .45 | .1 | .045 | | .9 | .45 | .1 | .045 | | .9 | .45 | .1 | .045 |
| hCG OmIU/ml | .027 | .027 | .029 | .028 | | .024 | .024 | .026 | .024 | | .020 | .016 | .022 | .023 |
| 50 mIU/ml (hCG) | .116 | .106 | .083 | .072 | | .117 | .111 | .100 | .099 | | .058 | .059 | .056 | .057 |
| LH 400 mIU/ml | .119 | .082 | .039 | .033 | | .135 | .091 | .056 | .05 | | .085 | .076 | .060 | .057 |
| % CRX | 13.0 | 8.8 | 2.3 | 1.5 | | 14.4 | 9.8 | 5.0 | 6.3 | | 21.0 | 17.4 | 13.9 | 12.5 |

## TABLE 3

| | 10 mM Tris | | | | | 50 mM Tris | | | | | 100 mM Tris | | | |
|---|---|---|---|---|---|---|---|---|---|---|---|---|---|---|
| KCl Concentration (%) | .9 | .45 | .1 | .045 | | .9 | .45 | .1 | .045 | | .9 | .45 | .1 | .045 |
| hCG OmIU/ml | .021 | .021 | .021 | .022 | | .020 | .021 | .019 | .023 | | .020 | .017 | .023 | .023 |
| 50 mIU/ml (hCG) | .073 | .073 | .044 | .040 | | .077 | .076 | .067 | .060 | | .060 | .062 | .059 | .056 |
| LH 400 mIU/ml | .082 | .066 | .028 | .026 | | .096 | .076 | .054 | .044 | | .083 | .076 | .060 | .056 |
| % CRX | 14.6 | 10.8 | 3.8 | 2.8 | | 16.6 | 12.5 | 9.1 | 7.1 | | 19.6 | 16.6 | 12.5 | 12.5 |

Example 4 (Simultaneous Procedure)

Utilizing the same components as earlier described under sequential immunoassay for hCG, LH cross-reaction was investigated using a simultaneous assay. Simultaneous assay is meant by the incubation of the antigen to be determined (LH or hCG) and labeled antibody to that antigen (enzyme conjugate), and allowing these reactants to bind to the anti-alpha-subunit coated tube at the same time. In this way the sandwich formation takes place during one incubation time. The remainder of the assay is identified.

In this system, the antigen is incubated with enzyme conjugate for 30 minutes at room temperature. Unbound antigen and enzyme conjugate are then washed away with a saline solution as set forth in Example 1. BCIP substrate is allowed to incubate until blue color develops. Enzyme reaction is then stopped and read as set forth in Example 1. The tris buffer was maintained at 50 mM. The results are set forth in Table 4.

TABLE 4

| Na Cl Conc. % | 0.9 | 0.45 | 0.1 | 0.045 |
|---|---|---|---|---|
| OmIU/ml hCG | .028 | .026 | .027 | .026 |
| 50mIU/ml hCG | .137 | .141 | .134 | .132 |
| 400mIU/ml LH | .141 | .124 | .088 | .073 |
| % CRX | 12.9 | 10.6 | 7.1 | 5.5 |

As with the sequential assay, LH cross-reactivity decreased with decreasing salt content in the simultaneous assay.

CLAIMS:

1. A sandwich-type immunoassay method for hCG in a biologically-derived fluid sample which includes hCG and LH, comprising the steps of

(a) contacting a solid phase comprising immobilized anti-alpha-subunit monoclonal antibody bound to a solid surface with the biologically-derived fluid sample, either before or substantially simultaneously with contacting with free labeled anti-beta-subunit monoclonal antibody specific for the beta subunit of hCG, to cause hCH and LH immunologically to bind to the immobilized antibody and an immunological reaction to occur linking the labeled antibody with bound hCG in solid phase, the labeled antibody being contained in a buffered solution having a concentration no greater than about 0.5 weight/volume % of potassium chloride, sodium chloride, or a mixture thereof;

(b) separating free unbound labeled antibody remaining after step (a); and

(c) detecting the bound labeled antibody as a measure of the quantity of hCG in the fluid sample.

2. A method as claimed in claim 1, in which the salt comprises sodium chloride.

3. A method as claimed in claim 1 or claim 2, in which the salt is present in the buffered solution at a concentration of from about 0.05 to about 0.5 weight/ volume %.

4. A method as claimed in any one of claims 1 to 3, in which the buffered solution includes a buffering agent at a concentration no greater than 100 mM.

5. A method as claimed in any one of claims 1 to 4, in which the buffered solution includes as a buffering agent a phosphate, tris, glycine, histidine borate, or a mixture thereof.

6. A method as claimed in any one of claims 1 to 5, in which the labeled antibody comprises an antibody-enzyme conjugate.

7. A method as claimed in any one of claims 1 to 5, in which the labeled antibody comprises a radioactively labeled, fluorometrically labeled or colorimetrically labeled antibody.

8. A method for reducing the cross-reactivity of LH for anti-beta-subunit monoclonal antibody specific for the beta subunit of hCG in an immunological reaction between hCG and the antibody when LH is also present, which method comprises performing the reaction in a buffer having a concetration of sodium and/or potassium chloride no greater than about 0.5 weight/volume % and/or the buffer having a concentration of buffering agent no greater than 100 mM.